# EUROPEAN PATENT SPECIFICATION

(11) **EP 2 389 900 B1**
(45) Date of publication and mention of the grant of the patent: **27.03.2013**
(21) Application number: 10163717.1
(22) Date of filing: 24.05.2010
(51) Int. Cl.: A61F 2/30

(54) **An implant for cartilage repair comprising an extending post**
Implantat für eine Knorpelreparatur mit einem abstehenden Bolzen
Implant pour réparation de cartilage comportant une tige étendue

(43) Date of publication of application: 30.11.2011
(73) Proprietor: Episurf Medical AB, 115 42 Stockholm (SE)
(72) Inventor: Bake, Nina, 181 90 LIDINGÖ (SE); Ryd, Leif, 113 40 STOCKHOLM (SE)
(74) Representative: Kitzler, Michael

(56) References cited:
- EP-A2- 1 277 450
- WO-A1-01/30276
- US-A1- 2007 093 896
- US-A1- 2008 249 632

## Description

### Field of the invention

This invention relates in general to the field of orthopedic implants. More particularly the present invention relates to a medical implant with a peg or extending post, for cartilage repair at an articulating surface in a joint such as a knee, hip, toe or shoulder.

### Background

### General background

Pain and overuse disorders of the joints of the body is a common problem. The weight-bearing and articulating surfaces of for example knees and other joints, are covered with a layer of soft tissue that typically comprises a significant amount of hyaline cartilage. The cartilage is prone to damage due to disease, injury or chronic wear and causes much suffering in terms of pain or disability to move freely. It is therefore important to have efficient means and methods for repairing damaged cartilage in knee joints. Large knee prostheses on the market are successful in relieving pain but there is a limit in the lifetime of the prostheses of 10-15 years.

These large prostheses have lead to the further development of smaller implants that can be implanted with less invasive surgery. In this development there has also been an effort to achieve small joint implants, suitable for repair of a small cartilage injury that have a minimal influence on the surrounding parts of the joint. In the current development such small implants are designed with an implant body that may be formed as a thin plate with a hard surface for facing the articulate side of the joint and a bone contacting surface for facing subchondral bone below the damaged part of cartilage. The shape and the curvature of the articulate surface of the implant may be designed to be similar to the shape and the curvature of the part of the joint where the implant is inserted. Such implants are designed with this implant body and may have a peg or a rod projecting from the bone contacting side of the implant body for fastening the implant to the bone, making them look like mushrooms.

### Specific background

In a surgical operation where small implants are inserted to replace damaged cartilage, it is critical that the implant is positioned in a precise manner and also that the implant can be smoothly positioned with no risks for misplacements. It is of great importance that the implant is inserted with the correct angle into the bone. If there is a small deviation from the planned insertion angle and direction there will be a mismatch between the implant and the surrounding cartilage, which can lead to further wear of surrounding cartilage areas. More specifically, if the implant is skewly positioned an edge of the implant may project over the surface of the cartilage, thereby being in risk of wearing the cartilage on the opposite surface of the joint. Also, the cartilage in the direct vicinity of the implant might be squeezed by the implant or out of contact with the implant, leading to less integration of the implant with the surrounding tissue. Today there is a need for short surgery times and also there is a need for less invasive surgery, such as small open surgery operations or laparoscopic surgery, both for convenience for the patients and for economical reasons. There is also a need of an implant with long life time in the cartilage, an implant which not is loosened from the bone after insertion.

There is a need for an improved implant which can be inserted into the implant site in a more correct and precise manner.

### Prior art

Examples of prior art which discloses smaller implants with anchoring means for cartilage replacement is found in the following patent publications.

US2003/0216669 A1 describes implants and mini prostheses with anchoring means in the shape of pegs which are larger than the drill hole where the pegs are to be placed and thus designed for pressfit, gives mechanical anchoring when hammered in place, due to the difference in size between peg and drill hole. The implants are fastened with a hammer (see figure 17C and [0200]).

WO03/05210 A2 describes implants with anchoring means in the shape of thread-shaped peg (see for example fig 3B).

W02008/101090 A2 describes implants with an anchoring mechanism in form of a peg (see fig 3).

WO2009/111626 A2 describes a method for designing an orthopedic device that alters a wear pattern on an articular surface of a joint. The pegs on the implants in this document are designed to facilitate the anchoring process [00123]. See fig 5B for example of anchoring peg.

US 2008/0249632 A1 describes a cartilage defect repair play comprising a top layer and a plurality of cylinder layers forming a stepped cone attached to the top layer.

### Object of the Invention

### General object

The general object of the invention is to solve the problem of providing an implant that facilitates the insertion of the implant in an articulate surface of a joint. In one aspect the object is to provide an implant which may be implanted in a correct position and with higher precision, thereby reducing risks of wear damage to surrounding cartilage and also improving integration of the implant with the surrounding tissue to give a permanent, solid attachment of the implant. In another aspect the object of the invention is to provide minimal anchoring (minimal trauma to surrounding bone and tissue) for saving bone for potential later prosthesis and other surgical alternatives. In still another aspect the object of the invention is to provide an improved implant which is easy for the surgeon to place in a joint.

### Summary of the invention

The present invention relates to an implant with a peg or extending post which has been designed to improve and facilitate easier and more precise attachment of the implant to the damaged joint. The extending post has a fixation part, proximal to the implant body, which is preferably designed to have a perfect fit to or slightly larger diameter than a drill hole in the bone to which it is to be attached. This ensures firm attachment of the extending post and the implant to the bone. In order to facilitate correct positioning and inclination of the implant, as it is to be hammered or screwed into the drill hole, the extending post is also provided with a positioning part, distal to the implant body. The positioning part has a cylindrical or cylindrical like shape with parallel side walls. It is also designed to have a diameter that is the same as or smaller than the diameter of the drill hole, such that it fits precisely to the drill hole, with a predetermined tolerance and play. This design ensures that the implant is correctly placed in relation to the drill hole, i.e. in correct position and angle.

A first aspect the invention is an implant for cartilage repair at an articulating surface of a joint. The implant comprises a contoured, substantially plate shaped implant body and at least an extending post, where the implant body has I) an articulate surface configured to face the articulating part of the joint; II) a bone contact surface configured to face the bone structure of a joint, where the fixation and bone contact surfaces face mutually opposite directions, and; III) an extending post, extending from the bone contact surface, that has a cylindrical shape, with a convex polygonal, circular or roughly circular, oval or irregular shape cross-section. The extending post comprises a fixation part that is proximal to the implant body and a positioning part that is distal to the implant body. The positioning part has a cylindrical shape, with a convex polygonal, circular or roughly circular, oval or irregular shape cross-section, and has a smaller diameter than the fixation part. The longitudinal symmetry axis of the fixation part and the positioning part coincide.

In one embodiment the invention further comprises a guide part at the distal tip of the positioning part, wherein the guide part has a smaller diameter than the positioning part.

Further varieties of the inventive concept comprise such an implant comprising any of the following optional individual or combinable aspects:
The length of the positioning part of the extending post is 1/10-1/2 of the length of the fixation part.
The positioning part of the extending post has a diameter which is 0,1-2 mm smaller than the diameter of the fixation part.
The diameter of the fixation part of the extending post is between 0.5-10 mm.
The diameter of the positioning part of the extending post is between 0.4-9 mm.
The length of the fixation part of the extending post is between 2-30 mm
The length of the positioning part of the extending post is between 1-15 mm.
The guiding part of the extending post is 1/10- 1/2 of the length of the fixation part.
The guiding part of the extending post is cylinder shaped, with a convex, polygonal or circular cross-sectional area.
The guiding part of the extending post is cone shaped and where the top of the cone is pointing away from the implant body.
The diameter of the guiding part of the extending post is between 0,4-5 mm.
The length of the guiding part of the extending post is between 1-10 mm.
The thickness of the implant body is between 1 mm and about 10 mm, preferably between about 2 mm and 6 mm.
The area of the articulate surface is between 0.5 cm² and 20 cm², between 0,5 cm² and 15 cm², between 0,5 cm² and 10 cm² or preferably between about 1 cm² and 10 cm².

One aspect the invention is a method for designing an implant for cartilage repair at an articulating surface of a joint, comprising the steps of
I) designing an implant that comprises a contoured, substantially plate shaped implant body and an extending post;
II) determining the diameter of a drill hole that is to be made in the bone of the joint, wherein the extending post is to be inserted;
III) designing said extending post to have a cylindrical shape, with a convex, polygonal or circular cross-sectional area, and to comprise a fixation part that is proximal to the implant body and a positioning part that is distal to the implant body, such that the longitudinal symmetry axis of the fixation part and the positioning part coincide;
IV) designing said fixation part to have a diameter that is larger than the diameter of the drill hole, to achieve press fit with a predetermined tolerance between the fixation part and the drill hole; and
V) designing said positioning part to have a diameter that is the same as or smaller than the diameter of the drill hole, such that the positioning part and the drill hole fit with a predetermined tolerance and play.

### Brief description of the figures

The present invention will be further explained below with reference to the accompanying drawings, in which:

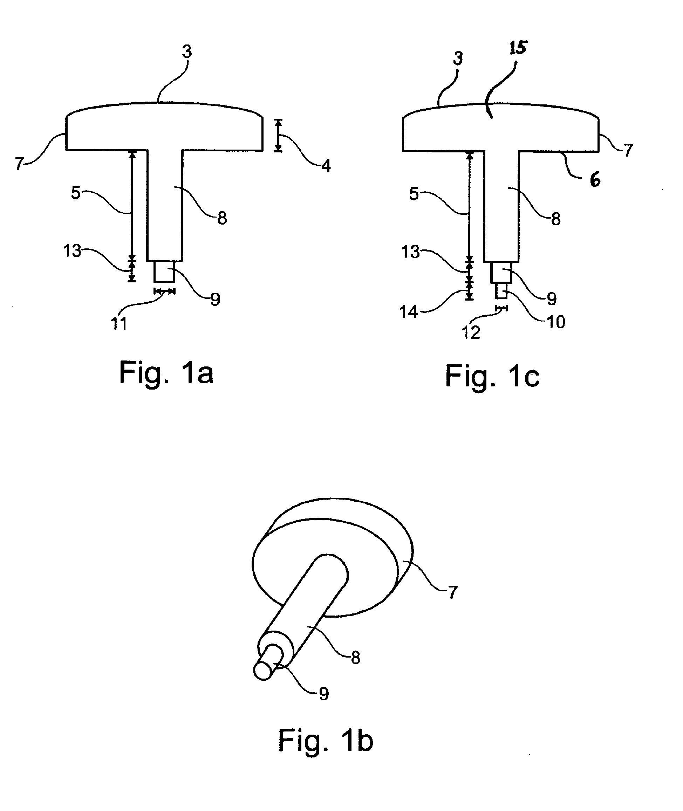

Fig 1a shows an exemplifying embodiment of an implant according to the present invention, showing a sectional view, with a substantially plate shaped body and an extending post with a positioning part.
Fig 1b shows an exemplifying embodiment of an implant according to the present invention, showing an angular view, with a substantially plate shaped body and an extending post with a positioning part.
Fig 1C shows another exemplifying embodiment of an implant according to the present invention, with a substantially plate shaped body and an extending post with a positioning part and a guide part.

### Detailed description of the invention

### Introduction

The invention concerns an implant which is intended to be used for replacing damaged cartilage in a joint, for example a knee, hip, shoulder or a toe. The implant is constructed in such a way that it facilitates both insertion and also optimizes the attachment and precision of fit for the implant in a drill hole, in which the implant is to be attached to the bone.

Figure 1 shows an embodiment of an implant according to the present invention. The implant comprises a substantially plate-shaped body 15 and an extending post 8, where said implant body 15 has an articulate surface 3, facing the articulating surface in a joint, and a bone contacting surface 6 facing the bone structure in the joint, and where the bone contacting surface 6 has an extending post 8. The extending post 8 has a fixation part 8, which is cylinder shaped and located proximal to the plate shaped implant body 15 and a positioning part 9, which also is cylinder shaped and located distal to the plate shaped implant body 15, at the end of the fixation part 8. The diameter 11 of the positioning part 9 is smaller than the diameter 2 of the fixation part 8. The positioning part 9 of the extending post 8 facilitates the insertion of the extending post 8 for the surgeon who performs the implant placement. As shown in figure 1C, the extending post 8 may in one embodiment have an additional guiding part 10 which extends from the end of the positioning part 9 of the extending post 8.

An implant according to the invention with the described construction or design solves the problem of misplacements and also facilitates the insertion of the implant for the surgeon, contributing to shorter surgery times.

### Details of the different parts of the implant

### The implant body

The implant 1 comprises a contoured, substantially plate shaped implant body 15. The implant body 15 has a thin, plate-like design meaning that its cross-sectional distance 18 is larger or even substantially larger than its thickness 4, e.g. at least 1.5 times larger. The plate can vary in size and shape and may be adjusted to the size and shape of the damaged cartilage tissue and to the needs of particular treatment situations. For instance the cross-sectional area of the implant body 15 may have a circular or roughly circular, oval, triangular, square or irregular shape. The size of the implant 1 may also vary. The surface area of the articulate surface 3 of the implant body 15 varies in different realizations of the invention between 0.5 cm² and 20 cm², between 0,5 cm² and 15 cm², between 0,5 cm² and 10 cm² or preferably between about 1 cm² and 10 cm². In general, small implants are preferred since they have a smaller impact on the joint at the site of incision and are also more easily implanted which leads to smaller open surgical procedures. The primary factor for determining the size of the implant is however the nature of the lesion in the cartilage to be repaired. The thickness of the implant body 15 is between 1 mm and about 10 mm, preferably between about 2 mm and 6 mm. The thickness of the implant should on the whole preferably match the thickness of the original cartilage layer, possibly also adapted to adjust for the recess in the bone, used for anchorage of the implant (see further explanation below) or formed as a part of the disease process.

### The extending post

The implant has an extending post 8 which extends from the bone contacting surface 6 of the implant body 15. In order to promote an immediate attachment of the implant to the bone as it is implanted into the body, the extending post 8 is used for immediate, mechanical attachment, called primary fixation.

The extending post 8 has a physical structure in the form of a circular, polygonal or roughly circular, oval or irregular shape cylinder. By polygonal cylinder is meant a geometrical form that is similar to a regular cylinder in that it has parallel side walls, but that has a cross sectional area that is polygonal, i.e. pentagonal, hexagonal, heptagonal or more. Preferably the polygonal cross-sectional area is convex and/or equiangular. Similarly, by roughly circular, oval or irregular shape cylinder is meant a geometrical form that is similar to a regular cylinder in that it has parallel side walls, but that has a cross sectional area that is any of a rough circle, an oval or an irregular shape. The cross-sectional area may be custom shaped for a specific patient. In a preferred embodiment the extending post 8 has the form of a circular or convex and/or equiangular polygonal cylinder.

The extending post 8 has a fixation part 8 and a positioning part 9, where the fixation part 8 is located proximal to the plate shaped implant body 15 and the positioning part 9 is located distal to the plate shaped implant body 15. The longitudinal symmetry axes of the fixation part 8 and the positioning part 9 coincide. The diameter of the positioning part 9 is smaller than the diameter of the fixation part 8. The positioning part 9 of the extending post 8 has a diameter 11 of 0,4-9 mm, preferably 0,6-5 mm. The diameter 2 of the fixation part 8 is between 0,5-10 mm, preferably 0,7-6 mm. In one embodiment the difference between the diameter of the fixation part 8 of an implant and the diameter of the positioning part 9 of the same implant is between 0,1-2 mm. In a specific embodiment the positioning part 9 has a diameter of 1,8 mm and the fixation part 8 has a diameter of 2 mm. The cross sectional area of the parts of the extension post can be circular, polygonal or roughly circular, oval or irregular shape cylinder. Within this text the meaning of the word diameter is thus to be interpreted as follows: the diameter of a circle is any straight line segment that passes through the center of the circle and whose endpoints are on the circle. The diameter of a polygon is the largest distance between any pair of vertices. In other words, it is the length of the longest polygon diagonal (i.e. straight line segment joining two vertices). The diameter of a roughly circular, oval or irregular shape cross-sectional area is the longest straight line segment that can be made which passes through the center of the cross sectional area and whose endpoints are on the circle.

The positioning part 9 of the extending post 8 has a length 13 of 1-15 mm and the length 5 of the fixation part 8 is between 2-30 mm. In one exemplifying embodiment, the length of the positioning part is shorter than the length of the fixation part, or preferably the length of the positioning part is 1/10-1/2 of the length of the fixation part.

In one embodiment the positioning part 9 of the extending post has a smooth or even surface with no protruding structures such as threads. In another embodiment both the fixation part 8 and the positioning part 9 of the extending post has a smooth or even surface with no protruding structures such as threads.

The implant is to be inserted during cartilage replacement surgery. During surgery the damaged cartilage and a surrounding area corresponding to the cross-sectional area of the implant body is first removed. A recess in the subchondral bone under the cartilage damage may also be removed e.g. by reaming, in order to facilitate more stable attachment of the implant to the joint. A drill hole is made in the bone at the position where the extending post is to be inserted. The implant is secured in the bone by the primary attachment of the extending post 8 in the drill hole in the bone. The implant design supports the surgeon by facilitating the placement of the implant. The positioning part 9 of the extending post 8 directs the surgeon when placing the implant 1 in the drill hole made in the bone. The cylinder shape, which is designed to fit more or less perfectly with the drill hole, ensures stable positioning and correct inclination of the extending post 8 and thus the implant. The fixation part 8 of the extending post 8 has a slightly larger diameter than the drill hole where it is to be inserted, in order to give a press fit between the fixation part 8 and the drill hole. Without the positioning part 9 of the extension 8 it is hard for the surgeon to find the drill hole and place the implant in the right place and angle. The positioning part 9 has the same diameter or a slightly smaller diameter than the drill hole. The positioning part 9 is used to make it easier for the surgeon to find the drill hole, and to place the implant in the right position and angle. The positioning part 9 of the extending post 8 can be placed in the drill hole by hand without using a hammer. Due to the precise fit between the positioning part 9 and the drill hole, the implant will automatically be placed in a correct and desired location and direction (angle). In addition, the implant will also have a solid support once placed or hammered into the drill hole, due to the press-fit that is gained from the diameter of the fixation part of the extending post being slightly larger than the diameter of the drill hole.

Compared to for example prior art implants with a cone shaped extending post, where only a small part of the cone has the same diameter as the drill hole, such implants give weaker attachment, since the contact area of the implant and the bone is smaller. A cone shaped extending post also is more difficult to place in the right angle and direction into the bone, since it may wiggle and twist a bit once placed in a drill hole.

In one exemplifying embodiment, see figure 1c, the extending post of the implant additionally has a guide part 10. The guide part 10 of the extending post 8 is also cylinder shaped, or alternatively cone shaped, and is located at the end of the positioning part 9, i.e. at the distal tip of the positioning part in relation to the implant body 15. The diameter 12 of the guide part is smaller than the diameter of the positioning part 11. The guide part 10 further facilitates insertion of the implant since the diameter of the guide part is smaller than the diameter of the drill hole. The guide part thus provides a first, initial guidance for the surgeon, by easily fitting into the drill hole. In the embodiments of the invention which have a guide part 10, the guide part 10 provides initial guidance to help the surgeon to find the drill hole. The positioning part 9 then serves as a positioning guide, by its precise fit with the drill hole, to give the right direction of the implant when the implant is hammered into the bone.

The longitudinal symmetry axis of the fixation part 8 and the positioning part 9 and the guiding part 10 coincide.

The guiding part 10 of the extending post 8 has a length 13 of 1-10 mm and a diameter of 0,4-5 mm, preferably 0,5-4 mm.

## Claims

1. A method for designing an implant (1) for cartilage repair at an articulating surface of a joint, comprising the steps of
designing an implant that comprises a contoured, substantially plate shaped implant body (15) and an extending post (8); and
determining the diameter of a drill hole that is to be made in the bone of the joint, wherein the extending post (8) is to be inserted
**characterized in that**
designing said extending post (8) to have a cylindrical shape, with a convex, polygonal or circular cross-sectional area, and to comprise a fixation part (8) that is proximal to the implant body (15) and a positioning part (9) that is distal to the implant body (15), such that the longitudinal symmetry axis of the fixation part (8) and the positioning part (9) coincide; and
designing said fixation part (8) to have a diameter (2) that is larger than the diameter of the drill hole, to achieve press fit with a predetermined tolerance between the fixation part (8) and the drill hole; and
designing said positioning part (9) to have a diameter (11) that is the same as or smaller than the diameter of the drill hole, such that the positioning part (9) and the drill hole fit with a predetermined tolerance and play.

2. A method for designing an implant (1) for cartilage repair according to any of the preceding claims wherein said length of the positioning part (9) of the extending post is 1/10-1/2 of the length of the fixation part (8).

3. A method for designing an implant (1) for cartilage repair according to any of the preceding claims wherein the positioning part (9) of the extending post (8) has a diameter (11) which is 0.1-2 mm smaller than the diameter (2) of the fixation part (8).

4. A method for designing an implant (1) for cartilage repair according to any of the preceding claims wherein the diameter of the fixation part (8) of the extending post (8) is between 0.5-10 mm.

5. A method for designing an implant (1) for cartilage repair according to any of the preceding claims wherein the diameter of the positioning part (9) of the extending post (8) is between 0.4-9 mm.

6. A method for designing an implant (1) for cartilage repair according to any of the preceding claims wherein the length of the fixation part (8) of the extending post (8) is between 2-30 mm

7. A method for designing an implant (1) for cartilage repair according to any of the preceding claims wherein the length of the positioning part (9) of the extending post (8) is between 1-15 mm.

8. A method for designing an implant (1) for cartilage repair according to any of the preceding claims wherein said implant (1) further comprises a guide part (10) at the distal tip of the positioning part (9), wherein the guide part (10) has a smaller diameter than the positioning part (9).

9. A method for designing an implant (1) for cartilage repair according to claim 8 wherein the length of the guiding part (10) of the extending post is 1/10-1/2 of the length of the fixation part (8).

10. A method for designing an implant (1) for cartilage repair according to claim 8-9 wherein the guiding part (10) of the extending post is cylinder shaped, with a convex, polygonal or circular cross-sectional area.

11. A method for designing an implant (1) for cartilage repair according to claim 8-10 wherein the guiding part (10) of the extending post (8) is cone shaped and where the top of the cone is pointing away from the implant body (15).

12. A method for designing an implant (1) for cartilage repair according to claim 8-11 wherein the diameter of the guiding part (10) of the extending post (8) is between 0,4-5 mm.

13. A method for designing an implant (1) for cartilage repair according to claim 8-12 wherein the length of the guiding part (10) of the extending post (8) is between 1-10 mm.

14. A method for designing an implant (1) for cartilage repair according to any of the preceding claims wherein the thickness (4) of the implant body (15) is between 1 mm and about 10 mm, preferably between about 2 mm and 6 mm.

15. A method for designing an implant (1) for cartilage repair according to any of the preceding claims wherein the area of the articulate surface (3) is between 0.5 cm² and 20 cm², between 0,5 cm² and 15 cm², between 0,5 cm² and 10 cm² or preferably between about 1 cm² and 10 cm².

## Patentansprüche

1. Verfahren zum Auslegen eines Implantats (1) zur Knorpelreparatur an einer Gelenkoberfläche eines Gelenks, umfassend die Schritte
Auslegen eines Implantats, das einen konturierten, im Wesentlichen plattenförmigen Implantatkörper (15) und einen vorstehenden Stift (8) umfasst; und
Bestimmen des Durchmessers eines Bohrlochs, dass in dem Knochen des Gelenks geschaffen werden soll, in das der vorstehende Stift (8) eingesetzt werden soll **gekennzeichnet durch**
Auslegen des vorstehenden Stifts (8), so dass er eine zylindrische Form mit einer konvexen, polygonalen oder kreisförmigen Querschnittsfläche aufweist, und um einen Befestigungsteil (8), der proximal zu dem Implantatkörper (15) ist, und einen Positionierungsteil (9) zu umfassen, der distal zu dem Implantatkörper (15) ist, so dass die longitudinale Symmetrieachse des Befestigungsteils (8) und des Positionierungsteils (9) übereinstimmen; und
Auslegen des Befestigungsteils (8), so dass er einen Durchmesser (2) aufweist, der größer ist als der Durchmesser des Bohrlochs, um eine Presspassung mit einer vorbestimmten Toleranz zwischen dem Befestigungsteil (8) und dem Bohrloch zu erhalten; und
Auslegen des Positionierungsteils (9), so dass es einen Durchmesser (11) aufweist, der gleich dem oder kleiner als der Durchmesser des Bohrlochs ist, so dass der Positionierungsteil (9) und das Bohrloch mit einer vorbestimmten Toleranz und einem vorbestimmten Spiel passen.

2. Verfahren zum Auslegen eines Implantats (1) zur Knorpelreparatur nach einem der vorhergehenden Ansprüche, wobei die Länge des Positionierungsteils (9) des vorstehenden Stifts 1/10-1/2 der Länge des Befestigungsteils (8) beträgt.

3. Verfahren zum Auslegen eines Implantats (1) zur Knorpelreparatur nach einem der vorhergehenden Ansprüche, wobei der Positionierungsteil (9) des vorstehenden Stifts einen Durchmesser (11) aufweist, der 0,1-2 mm kleiner ist als der Durchmesser (2) des Befestigungsteils (8).

4. Verfahren zum Auslegen eines Implantats (1) zur Knorpelreparatur nach einem der vorhergehenden Ansprüche, wobei der Durchmesser des Befestigungsteils (8) des vorstehenden Stifts (8) zwischen 0,5-10 mm liegt.

5. Verfahren zum Auslegen eines Implantats (1) zur Knorpelreparatur nach einem der vorhergehenden Ansprüche, wobei der Durchmesser des Positionierungsteils (9) des vorstehenden Stifts (8) zwischen 0,4-9 mm liegt.

6. Verfahren zum Auslegen eines Implantats (1) zur Knorpelreparatur nach einem der vorhergehenden Ansprüche, wobei die Länge des Befestigungsteils (8) des vorstehenden Stifts (8) zwischen 2-30 mm liegt.

7. Verfahren zum Auslegen eines Implantats (1) zur Knorpelreparatur nach einem der vorhergehenden Ansprüche, wobei die Länge des Positionierungsteils (9) des vorstehenden Stifts (8) zwischen 1-15 mm liegt.

8. Verfahren zum Auslegen eines Implantats (1) zur Knorpelreparatur nach einem der vorhergehenden Ansprüche, wobei das Implantat (1) weiter einen Führungsteil (10) an der distalen Spitze des Positionierungsteils (9) umfasst, wobei der Führungsteil (10) einen kleineren Durchmesser aufweist als der Positionierungsteil (9).

9. Verfahren zum Auslegen eines Implantats (1) zur Knorpelreparatur nach Anspruch 8, wobei die Länge des Führungsteils (10) des vorstehenden Stifts 1/10-1/2 der Länge des Befestigungsteils (8) beträgt.

10. Verfahren zum Auslegen eines Implantats (1) zur Knorpelreparatur nach Anspruch 8 bis 9, wobei der Führungsteil (10) des vorstehenden Stifts zylinderförmig mit einer konvexen, polygonalen oder kreisförmigen Querschnittsfläche ist.

11. Verfahren zum Auslegen eines Implantats (1) zur Knorpelreparatur nach Anspruch 8 bis 10, wobei der Führungsteil (10) des vorstehenden Stifts (8) kegelförmig ist und wobei die Oberseite des Kegels von dem Implantatkörper (15) weg weist.

12. Verfahren zum Auslegen eines Implantats (1) zur Knorpelreparatur nach Anspruch 8 bis 11, wobei der Durchmesser des Führungsteils (10) des vorstehenden Stifts (8) zwischen 0,4-5 mm liegt.

13. Verfahren zum Auslegen eines Implantats (1) zur Knorpelreparatur nach Anspruch 8 bis 12, wobei die Länge des Führungsteils (10) des vorstehenden Stifts (8) zwischen 1-10 mm liegt.

14. Verfahren zum Auslegen eines Implantats (1) zur Knorpelreparatur nach einem der vorhergehenden Ansprüche, wobei die Dicke (4) des Implantatkörpers (15) zwischen 1 mm und etwa 10 mm liegt, bevorzugt zwischen etwa 2 mm und 6 mm.

15. Verfahren zum Auslegen eines Implantats (1) zur Knorpelreparatur nach einem der vorhergehenden Ansprüche, wobei die Fläche der Gelenkfläche (3) zwischen 0,5 cm² und 20 cm² liegt, zwischen 0,5 cm² und 15 cm², zwischen 0,5 cm² und 10 cm², oder bevorzugt zwischen etwa 1 cm² und 10 cm².

## Revendications

1. Un procédé de conception d'un implant (1) destiné à la réparation du cartilage au niveau d'une surface d'articulation d'une articulation, comprenant les étapes consistant à :
concevoir un implant qui comprend un corps d'implant (15) profilé, sensiblement en forme de plaque, et une tige d'extension (8), et
déterminer le diamètre d'un trou de forage qui doit être fait dans l'os de l'articulation, dans lequel la tige d'extension (8) doit être insérée **caractérisé par** le fait de
concevoir ladite tige d'extension (8) de manière à ce qu'elle ait une forme cylindrique, avec une section convexe, polygonale ou circulaire en coupe transversale, et qu'elle comprennent une partie de fixation (8) qui est proximale par rapport au corps d'implant (15) et une partie de positionnement (9) qui est distale par rapport au corps d'implant (15), de telle sorte que l'axe de symétrie longitudinal de la partie de fixation (8) et de la partie de positionnement (9) coïncident, et
concevoir ladite partie de fixation (8) de manière à ce qu'elle ait un diamètre (2) qui soit plus grand que le diamètre du trou de perçage, afin d'obtenir une mise en place par pression, avec une tolérance prédéterminée entre la partie de fixation (8) et le trou de forage, et
concevoir ladite partie de positionnement (9) de manière à ce qu'elle ait un diamètre (11) qui soit le même que le diamètre du trou de forage, ou plus petit que ce diamètre, de telle sorte que la partie de positionnement (9) et le trou de forage s'épousent avec une tolérance et un jeu prédéterminés.

2. Un procédé pour la conception d'un implant (1) destiné à la réparation du cartilage selon l'une quelconque des revendications précédentes, dans lequel ladite longueur de la partie de positionnement (9) de la tige d'extension va de 1/10e à 1/2 de la longueur de la partie de fixation (8).

3. Un procédé pour la conception d'un implant (1) destiné à la réparation du cartilage selon l'une quelconque des revendications précédentes, dans lequel la partie de positionnement (9) de la tige d'extension (8) présente un diamètre (11) qui est de 0,1 à 2 mm plus petit que le diamètre (2) de la partie de fixation (8).

4. Un procédé pour la conception d'un implant (1) destiné à la réparation du cartilage selon l'une quelconque des revendications précédentes, dans lequel le diamètre de la partie de fixation (8) de la tige d'extension (8) est compris entre 0,5 et 10 mm.

5. Un procédé pour la conception d'un implant (1) destiné à la réparation du cartilage selon l'une quelconque des revendications précédentes, dans lequel le diamètre de la partie de positionnement (9) de la tige d'extension (8) est compris entre 0,4 et 9 mm.

6. Un procédé pour la conception d'un implant (1) destiné à la réparation du cartilage selon l'une quelconque des revendications précédentes, dans lequel la longueur de la partie de fixation (8) de la tige d'extension (8) est comprise entre 2 et 30 mm.

7. Un procédé de conception d'un implant (1) destiné à la réparation du cartilage selon l'une quelconque des revendications précédentes, dans lequel la longueur de la partie de positionnement (9) de la tige d'extension (8) est comprise entre 1 et 15 mm.

8. Un procédé pour la conception d'un implant (1) destiné à la réparation du cartilage selon l'une quelconque des revendications précédentes, dans lequel ledit implant (1) comprend en outre une partie de guidage (10) à l'extrémité distale de la partie de positionnement (9), la partie de guidage (10) présentant un diamètre plus petit que celui de la partie de positionnement (9).

9. Un procédé pour la conception d'un implant (1) destiné à la réparation du cartilage selon la revendication 8, dans lequel la longueur de la partie de guidage (10) de la tige d'extension (8) va de 1/10e à 1/2 de la longueur de la partie de fixation (8).

10. Un procédé de conception d'un implant (1) destiné à la réparation du cartilage selon les revendications 8 ou 9, dans lequel la partie de guidage (10) de la tige d'extension (8) est en forme de cylindre, avec une section convexe, polygonale ou circulaire en coupe transversale.

11. Un procédé de conception d'un implant (1) destiné à la réparation du cartilage selon l'une des revendications 8 à 10, dans lequel la partie de guidage (10) de la tige d'extension (8) est en forme de cône et la partie supérieure du cône est orientée à l'opposé du corps d'implant (15).

12. Un procédé de conception d'un implant (1) destiné à la réparation du cartilage selon l'une des revendications 8 à 11, dans lequel le diamètre de la partie de guidage (10) de la tige d'extension (8) est comprise entre 0,4 et 5 mm.

13. Un procédé de conception d'un implant (1) destiné à la réparation du cartilage selon l'une des revendications 8 à 12, dans lequel la longueur de la partie de guidage (10) de la tige d'extension (8) est comprise entre 1 et 10 mm.

14. Un procédé de conception d'un implant (1) destiné à la réparation du cartilage selon l'une quelconque des revendications précédentes, dans laquelle l'épaisseur (4) du corps d'implant (15) est comprise entre 1 mm et environ 10 mm, de préférence entre environ 2 mm et 6 mm.

15. Un procédé de conception d'un implant (1) destiné à la réparation du cartilage selon l'une quelconque des revendications précédentes, dans lequel l'aire de la surface articulée (3) est comprise entre 0,5 cm² et 20 cm², entre 0,5 cm² et 15 cm², entre 0,5 cm² et 10 cm² ou de préférence entre environ 1 cm² et 10 cm².
